# EUROPEAN PATENT APPLICATION

(11) **EP 1 466 894 A1**
(43) Date of publication of application: **13.10.2004**
(21) Application number: 04398003.6
(22) Date of filing: 07.04.2004
(51) Int. Cl.: C07C 279/02, C07C 277/08

(54) **Tetraalkyl-dimethylguanidinium salts and their use as ionic liquids**

(30) Priority: 09.04.2003 PT 10293703
(71) Applicant: Reis de Aguiar Navarro y Rosa, Joao Miguel, 7595 Torrao (PT); Almeida Fernandes Cobra Branco, Luis Alexandre, 1900 Lisboa (PT); Mateus Afonso, Carlos Alberto, 2815-752 (PT); Pimenta Gois, Pedro Miguel, 1170-270 (PT)
(72) Inventor: Magalhaes Mateus, Nuno Miguel, 7595 Torrao (PT); Almeida Fernandes Cobra Branco, Luis Alexandre, 1900 Lisboa (PT); Mateus Alfonso, Carlos Alberto, 2815-752 (PT); Reis de Aguiar Navarro Y Rosa, Joao Miguel, 7595 Torrao (PT); Pimenta Gois, Pedro Miguel, 1170-270 (PT); Torres Lourenso, Nuno Miguel, 7595 Torrao (PT)

(57) **Abstract**

This invention relates to the production and characterization of a series of tetra-alkyl-dimethylguanidinium salts as well as to its synthetic method and potential use as an ionic alternative medium to traditional volatile organic solvents. The synthetic method for these compounds involves a reaction between N,N-dimethyl-phosgeniminium chloride and several dialkylamines. Obtaining liquid salts, which melt below or just above room temperature, is generally achieved by anion exchange. The main innovation of this invention consists of using the full range of guanidine derivatives as reactional medium either for organic, inorganic or organometallic transformations.

## Description

### Background of Art

This invention consists of the description and characterization of a series of tetra-alkyl-dimethylguanidinium salts including their synthesis and their potential use as effective alternative solvents to traditional volatile organic solvents.

Usually these alternative solvents are called room temperature ionic liquids (abbreviated to RTIL) being compounds consisting entirely of ions species with an organic cation and an inorganic or organic anion (see for example T. Welton, *Chem. Rev.* 1999, **99**, 2071).

The field of application of this invention is Chemistry and Biology, namely in chemical or biological reactional processes conducted in ionic liquids.

The industrial domains for this application include general industrial chemistry, fine chemicals production, bioconversion processes in Biotechnology, environmental protection or bioremediation, the nanotechnology area and technological processes involving separation or transport of compounds through membranes.

The traditional solvents so commonly used in chemical industry present serious risks to the environment as well as to public health. The amount of solvent necessary in synthetic processes and other purification or extraction methods, associated with its high volatility, makes its containment, storage and reutilization difficult.

Recently the arising of the concept of environmentally sustainable chemical industry has helped to increase the research on "greener" alternatives to traditional organic solvents.

Ionic liquids (RTILs) have intrinsically useful properties, such as thermal stability (over 300°C), high ionic conductivity, negligible vapour pressure and a large electrochemical window. Depending on the anion and alkyl group of the imidazolium cation, RTILs can solubilize carbonyl compounds, alcohols, alkyl halides, supercritical CO2 (scC02) and also transition metal complexes. Simultaneously, they demonstrate low miscibility with water, alkanes and dialkyl ethers.

Applications of RTILs (Room Temperature Ionic Liquids) include their use as a recyclable media for chemical processes, including bio- and chemical catalysis, CO₂ capture, biphasic extraction, gas chromatography, selective transport using supported liquid membranes, pervaporation, dissolution of cellulose, in fuel cells, and their potential use as a matrix for mass spectrometry or as a solvent in nuclear electrical power plants.

Examples in bio- and chemical catalysis applications are described in R. Sheldon, *Chem. Commun.* 2001, 2399; C. M. Gordon, *Applied Catalysis A: General* 2001, **222**, 101 and P. Wasserscheid, W. Keim, *Angew. Chem. Int. Ed* 2000, **39**, 3772.

Examples in biphasic extraction applications are described in J. Dupont, C. S. Consorti, J. Spencer, *J. Braz. Chem. Soc.* 2000, **11**, 337. d) J. F. Brennecke, E. J. Maginn *AIChE Journal* 2001, **47**, 2384.

Examples in supported liquid membrane applications are described in L. C. Branco, J. G. Crespo, C. A. M. Afonso, *Angew. Chem. Int. Ed* 2002, **41**, 2771. b) L. C. Branco, C. A. M. Afonso, *Chem. Eur. J.* 2002, **8**, 3865. c) A. Aggarwal, N. L. Lancaster, A. R. Sethi, T. Welton *Green Chem.* 2002, **4**, 517.

Examples in other applications are E. D. Bates, R. D. Mayton, I. Ntai, J. H. Davis Jr, *J. Am. Chem. Soc.* 2002, **124,** 926, T. Schäfer, C. M. Rodrigues, C. A. M. Afonso, J. G. Crespo, *Chem. Commun.,* 2001, 1622.R. P. Swatloski, S. K. Spear, J. D. Holbrey, R. D. Rogers, *J. Am. Chem. Soc.* 2002, **124,** 4974, D. Allen, G. Baston, A. E. Bradley, T. Gorman, A. Haile, I. Hamblett, J. E. Hatter, M. J. F. Healey, B. Hodgson, R. Lewin, K. V. Lovell, B. Newton, W. R. Pitner, D. W. Rooney, D. Sanders, K. R. Seddon, H. E. Sims, R. C. Thied, *Green Chem.* 2002, **4**, 152, and references cited therein.

The use of ionic liquids as a recyclable and environmentally benign medium has been attracting considerable attention for chemical transformations including non-catalytic reactions, biocatalytic and catalytic reactions in monophasic systems (both substract and catalyst dissolved in the ionic liquid, and sometimes the ionic liquid works like a catalyst itself), biphasic systems (with the catalyst dissolved in the ionic liquid and the substract/product in a second phase or vice versa) and triphasic systems( with an ionic liquid phase, an organic phase and an aqueous phase).

Reactions like oxidations, hydrogenations, hydroformylations, Heck reaction, olefin oligomerisation, Trost-Tsuji coupling, dihydroxylations and epoxidations among several others have been tried successfully using such systems.

Solute extraction and recovery using supported liquid membranes is recognized as one of the most promising membrane-based processes. In a supported liquid membrane system an adequate solvent or solvent/carrier solution is immobilized inside the porous structure of a polymeric or ceramic membrane, in such a way that the feed phase, in which the solutes of interest are solubilized, is separated from the receiving phase, where these solutes are transferred to and, eventually, concentrated.

This configuration has attracted a great deal of interest, but its industrial application has been hindered by the difficulty in designing supported liquid membranes that exhibit high stability during operation.

The use of room-temperature ionic liquids (RTILs) as an immobilized phase in a supporting membrane is particularly interesting due to the nonvolatile character of the RTILs and their solubility properties in the surrounding phases, which makes it possible to obtain very stable supported liquid membranes without any observable loss of the RTIL to the atmosphere or the contacting phases.

The properties of RTILs are generally dependent on both ion structures. Regarding the cation, there are several reported examples consisting of alkyl phosphonium, ammonium (including chiral ones), sulfonium, pyridinium and 8-alkyl-1,8-diazabicyclo[5,4,0]-7-undecenium [alkyl-DBU], although the 1,3-dialkyl-imidazolium [im] unit is the most commonly used cation.

For the anion a broad range of examples have been reported, ranging from air-sensitive Lewis acids to more stable anions such as ClO₄⁻, NO₃⁻, BF₄⁻, PF₆⁻, alkyl and aryl sulfonates, phosphates, carboxylates and even more inert carboranes.

In spite of the considerable stability and lower viscosity of the RTILs based on the [im] unit, the presence of the more labile proton H-C(2) can be useful in some cases, due to formation of strong hydrogen bonds, but, depending on the anion used, can also react with electrophiles under basic conditions.

### Description of the invention

The main innovation of this invention consists of using the full range of guanidine derivatives as reactional medium either for organic, inorganic or organometallic transformations.

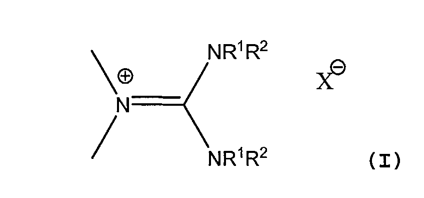

Compounds (I) are based on a tetra-alkyl-dimethylguanidinium cation having the general formula [(CH₃)₂N=C(NR¹R²)₂]⁺, and an organic or inorganic anion X⁻.

In general structure (I), R1 and R2 are, independently for each occurrence, identical or different and are each individually:
a) H, F, Cl, OH, SH or NH₂,
b) an hydrocarbyl radical (C1 to C12) that may have one or more double or triple bonds, and may include one or more saturated, unsaturated or aromatic rings,
c) a radical as in b), where 1 to 6 CH₂ units are replaced by di-radicals which are the same or different and may be 0, NR³, S, SO, SO₂, CO, SiR³R⁴ or P(O)R³, wherein R³ and R⁴ are, independently for each occurrence, radicals as defined in a) to e),
d) a radical as in b) or c), where 1 to 6 CH units are replaced by tri-radicals which are the same or different and may be N, SiR⁵, or PO, wherein R⁵ is, independently for each occurrence, a radical as defined in a) to e),
e) a radical as in b), c) or d), wherein one or more hydrogen atoms are replaced by F, Cl, Br, I, OH, SH or NH₂;
any 2 R¹ and/or R² above may be bonded together by one or more single, double or triple bonds, forming one or more rings, including aromatic rings.

The anion X⁻ in (I) can be
(i) an inorganic anion comprising:
   a) halides (F⁻, Cl⁻, Br⁻, I⁻) , phosphites and phosphates (PF₆-, H₂PO₂⁻ , H₂PO₄⁻ , PO₃⁻) , borates (BF4-, BO2-, BO3-), nitrites and nitrates (NO₂⁻, NO₃⁻) , sulfates (HSO₄⁻), cyanide and cyanates (CN⁻, SCN⁻, CNO⁻) , dicyanamide ((CN)₂N⁻), azide (N₃⁻) , carbonates (HCO₃⁻), bromate(BrO₃⁻), iodates (IO₃⁻, IO₄⁻), chlorites and chlorates (ClO⁻, ClO₂⁻, ClO₃⁻, ClO₄⁻) ;
   b) metal halide anions, comprising ZnCl₃⁻, ZnBr₃⁻, SnCl₅⁻, SnBr₅⁻, FeCl₄⁻, FeBr₄⁻, NiCl₃⁻, AuCl₄⁻, AuBr₄⁻, GaCl₄⁻, AlCl₄⁻, Al₂Cl₇⁻, Al₃Cl₁₀⁻ ;
   c) anions containing other chemical elements, preferably from groups Ib to VIIb, groups IIIa to VIa and group VIII from the periodic table, more preferably containing the elements arsenic (AsF₆⁻, H₂AsO₃⁻, H₂AsO₄⁻, AsO₂⁻), antimony (SbF₆⁻, SbO₃⁻), chromium (HCrO₄⁻) , tellurium (HTeO₃⁻ , HTeO₄⁻) , selenium (HSeO₃⁻ , SeCN⁻) , niobium (NbO₃⁻) , thallium (TaO₃⁻), ruthenium (RuO₄⁻) , manganese (MnO₄⁻) , rhenium (ReO₄⁻) , bismuth (BiO₃⁻), vanadium (VO₄⁻) and silver (Ag (CN)₂) ⁻;
(ii) an organic anion comprising:
   a) carboxylates, thiocarboxylates, carbamates, dithiocarbamates, xanthates, sulfonates, organo-sulfates, organo-sulfamates, organo-phosphates, phosphonates, thiophosphonates and other anions having the general formula R¹-Z-Y⁻ (II), wherein
      Z is SO₂, P(Y)R², As(Y)R² or CY,
      Y is, independently for each occurrence, O or S and
      R¹ and R² are identical or different and are each individually:
      a1) H, F, Cl, OH, SH or NH₂,
      a2) an hydrocarbyl radical (C1 to C30) that may have one or more double or triple bonds, and may include one or more saturated, unsaturated or aromatic rings,
      a3) a radical as in a2), where 1 to 15 CH₂ units are replaced by di-radicals which are the same or different and may be O, NR³, S, SO, SO2, CO, SiR³R⁴ or P (O) R³, wherein R³ and R⁴ are, independently for each occurrence, radicals as defined in a1) to a5),
      a4) a radical as in a2) or a3), where 1 to 15 CH units are replaced by tri-radicals which are the same or different and may be N, SiR⁵, or PO, wherein R⁵ is, independently for each occurrence, a radical as defined in a1 to a5,
      a5) a radical as in a2) a3) or a4), wherein one or more hydrogen atoms are replaced by F, Cl, Br, I, OH, SH or NH₂;
      R¹ and R² above may be bonded together by one or more single, double or triple bonds, forming one or more rings, including aromatic rings;
   b) Imides, thioimides, sulfonimides, N-acyl-sulfonamides, N-acil-phosphoramides and other anions having the general structure

      (R¹-Z-)₂N⁻ **(II)**

      wherein
   Z is, independently for each occurrence, SO₂, P(Y)R², As (Y) R² or CY,
   Y is, independently for each occurrence, O or S and
   R¹ and R² are, independently for each occurrence, identical or different and are each individually radicals as defined in a1) to a5); any pair of R¹ and/or R² may be bonded together by one or more single, double or triple bonds, forming one or more rings, including aromatic rings;
c) ascorbates, isocyanurates, barbiturates, ferrocenecarboxylates, methanefullerenecarboxylates and mixtures of the anions in a) and b).

Scheme (IV) illustrates the synthetic mechanism of tetra-alkyl-dimethylguanidinium salts.

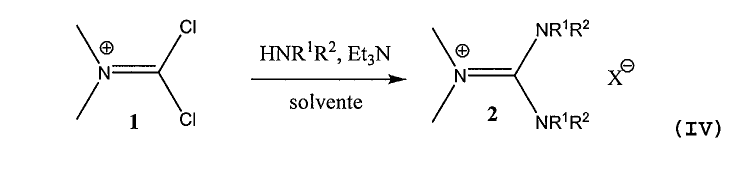

The conditions used consist of a one pot procedure starting from N,N-dimethyl-phosgeniminium choride (1) and several di-alkylamines. Two methods are described (A and B) according to the solubility properties of the resulting salts.

**General procedure for the preparation of guanidinium chlorides (Method A)** To a suspension of phosgene iminium chloride **(IV)**1 in a selected organic solvent preferably anhydrous dichloromethane at temperature between -20°C and 10°C, a mixture of secondary amine and triethylamine is added dropwise, in the same solvent. After 30 to 90 minutes preferably between -20°C and 15°C, stirring is maintained until completion of the reaction (complete solubilization of the phosgeniminium salt). The solvent is removed under vacuum and an aqueous solution of NaOH is added, with vigorous stirring. The mixture is washed with an organic solvent preferably diethyl ether and the pH is adjusted to 7 by addition of hydrochloric acid. The water is removed under vacuum, the residue extracted with an organic solvent preferably dichloromethane and dried (MgSO₄) . Finally, the solvent is evaporated and the residue left under vacuum (< 1 mm Hg) during 24 to 48 hours. The guanidinium salt **(IV)2** is obtained as a solid or as an oil.

**General procedure for the preparation of guanidinium chlorides (Method B)** To a suspension of phosgene iminium chloride **(IV)**1 in a selected organic solvent preferably anhydrous dichloromethane at temperature between -20 °C and 10°C, a mixture of secondary amine and triethylamine is added dropwise, in the same solvent. After 30 to 90 minutes preferably between -20°C and 15°C, stirring is maintained until completion of the reaction (complete solubilization of the phosgeniminium salt). The solvent is removed under vacuum and water is added. The aqueous phase is extracted with organic solvent preferably diethyl ether. The combined organic layers are washed with hydrochloric acid aqueous solution. The solvent is removed under vacuum, the residue extracted with organic solvent preferably dichloromethane and dried (MgSO₄). Finally, the solvent is evaporated and left under vacuum (< 1 mmHg) during 24 to 48 hours. The guanidinium salt **(IV)**2 is obtained as a solid or as an oil.

**General procedure for the substitution of chloride with inorganic anions:** The tetra-alkyl-dimethylguanidinium chloride (IV)2 is dissolved in organic solvent preferably dichloromethane. The desired anion is then added as a salt (for example KPF₆, NaBF₄ or LiNTf₂) or as an acid (for example HPF₆ or HBF₄) and the mixture is stirred at room temperature for 24 to 48 hours. The resulting solid is collected by filtration and washed with an organic solvent preferably dichloromethane. The combined organic layers are collected, dried (MgSO₄) and filtered.

**General procedure for the substitution of chloride with organic anions:** The desired organic anion is added as a salt or as an acid (for example mandelic acid, quinic acid, camphorsulfonic acid, aminoacids and protected aminoacids with N-BOC and N-acetyl groups) to the tetra-alkyl-dimethylguanidinium chloride **(IV)**2 dissolved in organic solvent preferably dichloromethane. When the organic anion is added as an acid, it is firstly transformed in a salt by addition of equimolar NaOH solution in organic solvent preferably methanol or ethanol. The reactional mixture is stirred at room temperature for 24 to 48 hours. The resulting solid is collected by filtration and washed with organic solvent preferably dichloromethane. The combined organic layers are collected, dried (MgSO₄) and filtered.

The following examples illustrate the procedure described above of according with two methods (A and B)

### Example 1

### [bis-(dibutylamino)-dimethylguanidinium] chloride synthesis

To a suspension of phosgene iminium chloride (2.14 g) in anhydrous dichloromethane (0.3M) at 0°C, a mixture of secondary amine and triethylamine, a mixture of dibutylamine (3.58 g) and triethylamine (2.93 g), in dichloromethane. After 30 minutes at 0 °C, vigorous stirring was maintained at room temperature during 3.5 hours. The solvent was removed under vacuum, water (50 mL) and an aqueous solution of NaOH (1.31 g) were added, with vigorous stirring. The mixture was extracted with diethyl ether (2 x 50 mL) and the pH was adjusted to 7 by addition of hydrochloric acid. The water was removed under vacuum. The guanidinium salt was obtained as a yellow coloured hygroscopic solid. (yield: 81%; 3.70 g)

### Example 2

### [bis-(ethylbutylamino)-dimethylguanidinium] chloride synthesis

To a suspension of phosgene iminium chloride (14.07 g) in anhydrous dichloromethane (0.3M) at 0°C, a mixture of ethylbutylamine (18,40g) and triethylamine (19.27 g) was added dropwise, in dichloromethane. After 30 minutes at 0 °C, vigorous stirring was maintained at room temperature during 3.5 hours. The solvent was removed under vacuum; water (150 mL) and an aqueous solution of NaOH (8.66 g) were added, with vigorous stirring. The mixture was extracted with diethyl ether (3 x 100 mL) and the pH was adjusted to 7 by addition of hydrochloric acid. The water was removed under vacuum. The guanidinium salt was obtained as a yellow coloured hygroscopic solid. (yield: 91%; 23.07 g)

### Example 3

### [bis-(dihexylamino)-dimethylguanidinium] chloride synthesis

To a suspension of phosgene iminium chloride (4.83 g) in anhydrous dichloromethane (0.3M) at 0°C, a mixture of dihexylamine (11.57 g) and triethylamine (6.61 g), in dichloromethane. After 30 minutes at 0 °C, vigorous stirring was maintained at room temperature during 3.5 hours. The solvent was removed under vacuum; water (75 mL) and an aqueous solution of NaOH (2.97 g) were added, with vigorous stirring. The mixture was extracted with diethyl ether (2 x 75 mL). The combined organic layers are washed with hydrochloric acid aqueous solution and dried (MgSO₄). Finally, the solvent is evaporated and the guanidinium salt was obtained as a orange coloured liquid. (yield: 86 %; 11.70 g)

### Example 4

### [bis-(dioctylamino)-dimethylguanidinium] chloride synthesis

To a suspension of phosgene iminium chloride (5.00 g) in anhydrous dichloromethane (0.3M) at 0°C, a mixture of dioctylamine (15.60 g) and triethylamine (6.85 g), in dichloromethane. After 30 minutes at 0 °C, vigorous stirring was maintained at room temperature during 3.5 hours. The solvent was removed under vacuum; water (100 mL) and an aqueous solution of NaOH (3.07 g) were added, with vigorous stirring. The mixture was extracted with diethyl ether (2 x 100 mL). The combined organic layers are washed with hydrochloric acid aqueous solution and dried (MgSO₄). Finally, the solvent is evaporated and the guanidinium salt was obtained as a orange coloured liquid. (yield: 70 %; 12.30 g) Some of the guanidinium salts prepared and several observed properties are presented in Table 1.

**Table 1.**

| Representative properties of some guanidinium salts. | | | | | | |
|---|---|---|---|---|---|---|
| Salt | Physical state^{a} | Miscible^{b} | Immiscible^{b} | Density^{c} | T_{g} (°C)^{d} | water content^{e} |
| 2a | Solid | dcm, H₂O, EtOH | Et₂O, hex | | -55.3 | |
| 3a | Solid | dcm, EtOH | Et₂O, H₂O, hex | | 56(mp) | |
| 4a | Solid | dcm, EtOH | Et₂O, H₂O, hex | | -57.7 | |
| 2b | Solid | dcm, H₂O, EtOH, | Et₂O, hex | | | |
| 3b | Liquid | dcm, EtOH | Et₂O, H₂O, hex | | | 9.4 |
| 4b | Solid | dcm, H₂O, EtOH | Et₂O, hex | | 51 (mp) | |
| 2c | Solid | dcm, H₂O, EtOH | Et₂O, hex | | -79.0 | |
| 3c | Solid^{f} | dcm, EtOH | Et₂O, H₂O, hex | | -67.8 | |
| 4c | Liquid | dcm, H₂O, EtOH | Et₂O, hex | 1.05 | | |
| 5c | Liquid | dcm, EtOH | Et₂O, H₂O, hex | 1.36 | -75.1 | 2.7 |
| 2d | Liquid | dcm, H₂O, EtOH, hex | Et₂O | 0.90 | -63.0 | 37.2 |
| 3d | Liquid | Et₂O, dcm, EtOH | H₂O, hex | | -60.5 | 2.9 |
| 4d | Liquid | Et₂O, dcm, EtOH | H₂O, hex | 0.97 | -75.6 | 15.6 |
| 5d^{g} | Liquid | dcm, EtOH | Et₂O, H₂O, hex | 1.20 | -71.4 | 4.5 |
| 2e | Liquid | Et₂O, dcm, EtOH, hex | H₂O | 0.96 | -76.0 | 47.8 |
| 3e | Liquid | Et₂O, dcm, EtOH | H₂O, hex | 0.91 | -78.0 | 3.9 |
| 4e | Liquid | Et₂O, dcm, EtOH | H₂O, hex | 0.97 | -75.3 | 7.6 |

| | | | | | | |
|---|---|---|---|---|---|---|
| ^{*a*}Physical state of the salt at room temperature | | | | | | |
| ^{*b*}Visually observed miscibility or immiscibility in water and in common organic solvents: hex = *n*-hexane, dcm = dichloromethane. | | | | | | |
| ^{*c*} Observed density (g.mL⁻¹). | | | | | | |
| ^{*d*} *T*_{*g*} = glass transition temperature | | | | | | |
| ^{*e*} Water content in µL (H ₂O).mL⁻¹ (RTIL). | | | | | | |
| ^{*f*} Liquid at 35 °C. | | | | | | |
| ^{*g*} Viscosity (cP): 346 (25°C), 335 (30°C), 296 (35°C), 269 (40°C), 182 (50°C), 124 (60°C). | | | | | | |

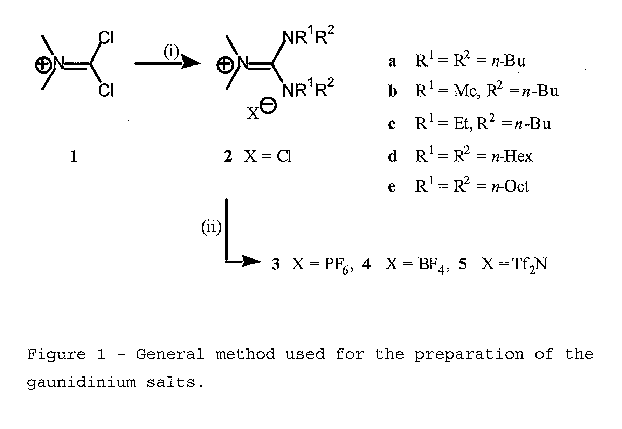

## Claims

1. A series of compounds **characterized by** the presence of tetra-alkyl-dimethylguanidinium unit [ (CH₃)₂N=C (NR¹R²) (NR³R⁴)] ⁺ **(I)** , and an organic or inorganic anion X⁻.

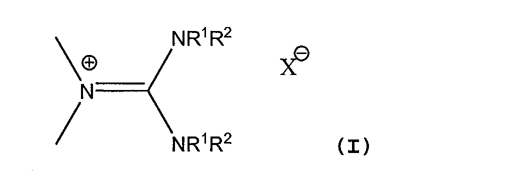

2. Compounds according to claim 1 **characterized by** the presence of tetra-alkyl-dimethylguanidinium unit **(I)**, and an organic or inorganic anion X⁻, chiral or achiral.

3. Compounds according to claims 1 and 2, wherein the anion X⁻ in **(I)** is
(i) an inorganic anion comprising:
a) halides (F⁻, Cl⁻, Br⁻, I⁻) , phosphites and phosphates (PF₆⁻, H₂PO₂⁻, H₂PO₄⁻, PO₃⁻) , borates (BF4-, BO2-, BO3-), nitrites and nitrates (NO₂⁻, NO₃⁻) , sulfates (HSO₄⁻), cyanide and cyanates (CN⁻, SCN⁻, CNO⁻) , dicyanamide ( (CN) ₂N⁻) , azide (N₃⁻) , carbonates (HCO₃⁻) , bromate(BrO₃⁻), iodates (IO₃⁻, IO₄⁻) , chlorites and chlorates (ClO⁻, ClO₂⁻, ClO₃⁻, ClO₄⁻) ;
b) metal halide anions, comprising ZnCl₃⁻, ZnBr₃⁻, SnCl₅⁻, SnBr₅⁻, FeCl₄⁻, FeBr₄⁻, NiCl₃⁻, AuCl₄⁻, AuBr₄⁻, GaCl₄⁻, AlCl₄⁻, Al2Cl₇⁻, Al₃Cl₁₀⁻ ;
c) anions containing other chemical elements, preferably from groups Ib to VIIb, groups IIIa to VIa and group VIII from the periodic table, more preferably containing the elements arsenic (AsF₆⁻, H₂AsO₃⁻, H₂AsO₄⁻, ASO₂⁻) , antimony (SbF₆⁻, SbO₃⁻) , chromium (HCrO₄⁻) , tellurium (HTeO₃⁻, HTeO₄⁻) , selenium (HSeO₃⁻, SeCN⁻) , niobium (NbO₃⁻) , thallium (TaO₃⁻) , ruthenium (RuO₄⁻) , manganese (MnO₄⁻), rhenium (ReO₄⁻) , bismuth (BiO₃⁻), vanadium (VO₄⁻) and silver (Ag (CN)₂) ⁻;
(ii) an organic anion comprising:
a) carboxylates, thiocarboxylates, carbamates, dithiocarbamates, xanthates, sulfonates, organo-sulfates, organo-sulfamates, organo-phosphates, phosphonates, thiophosphonates and other anions having the general formula R¹-Z-Y⁻ (II), wherein
Z is SO₂, P(Y)R², As(Y)R² or CY,
Y is, independently for each occurrence, O or S and
R¹ and R² are identical or different and are each individually:
a1) H, F, Cl, OH, SH or NH₂,
a2) an hydrocarbyl radical (C1 to C30) that may have one or more double or triple bonds, and may include one or more saturated, unsaturated or aromatic rings,
a3) a radical as in a2), where 1 to 15 CH₂ units are replaced by di-radicals which are the same or different and may be 0, NR³, S, SO, SO2, CO, SiR³R⁴ or P (O) R³, wherein R³ and R⁴ are, independently for each occurrence, radicals as defined in a1 to a5,
a4) a radical as in a2) or a3), where 1 to 15 CH units are replaced by tri-radicals which are the same or different and may be N, SiR⁵, or PO, wherein R⁵ is, independently for each occurrence, a radical as defined in a1) to a5),
a5) a radical as in a2), a3) or a4), wherein one or more hydrogen atoms are replaced by F, Cl, Br, I, OH, SH or NH₂;
R¹ and R² above may be bonded together by one or more single, double or triple bonds, forming one or more rings, including aromatic rings;
b) Imides, thioimides, sulfonimides, N-acyl-sulfonamides, N-acil-phosphoramides and other anions having the general structure
(R¹-Z-)₂N⁻ (II)
wherein
Z is, independently for each occurrence, SO₂, P(Y)R², As(Y)R² or CY,
Y is, independently for each occurrence, O or S and
R¹ and R² are, independently for each occurrence, identical or different and are each individually radicals as defined in a1 to a5;any pair of R¹ and/or R² may be bonded together by one or more single, double or triple bonds, forming one or more rings, including aromatic rings;
c) ascorbates, isocyanurates, barbiturates, ferrocenecarboxylates, methanefullerenecarboxylates and mixtures of the anions in a) and b).

4. Compounds according to claims 1, 2 and 3 synthesized from N,N-dimethyl-phosgeniminium salts **(IV)**1 .

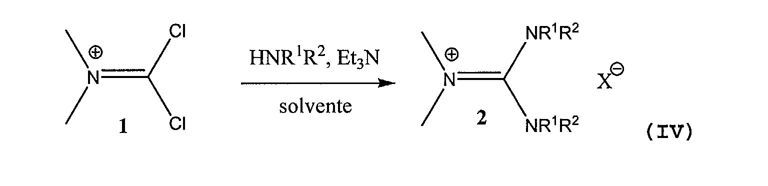

5. Compounds according to claims 1 to 4, synthesised from dialkylamines optionally containing one or more ester, ether, amine, aldehyde, ketone or carboxylic groups, specifically compounds with general structure (I), wherein R1 and R2 are, independently for each occurrence, identical or different and are each individually:
a) H, F, Cl, OH, SH or NH₂,
b) an hydrocarbyl radical (C1 to C12) that may have one or more double or triple bonds, and may include one or more saturated, unsaturated or aromatic rings,
c) a radical as in b), where 1 to 6 CH₂ units are replaced by di-radicals which are the same or different and may be O, NR³, S, SO, SO2, CO, SiR³R⁴ or P(O)R³, wherein R³ and R⁴ are, independently for each occurrence, radicals as defined in a) to e),
d) a radical as in b) or c), where 1 to 6 CH units are replaced by tri-radicals which are the same or different and may be N, SiR⁵, or PO, wherein R⁵ is, independently for each occurrence, a radical as defined in a) to e),
e) a radical as in b), c) or d), wherein one or more hydrogen atoms are replaced by F, Cl, Br, I, OH, SH or NH₂;
any 2 R¹ and/or R² above may be bonded together by one or more single, double or triple bonds, forming one or more rings, including aromatic rings.

6. Compounds according to claims 1 to 5 synthesized from chiral di-alkylamines.

7. The use of compounds according to claims 1 to 6 as solvent in supported liquid membranes applied to the selective transport of organic molecules (such as alcohols, amines, ethers, esters, carboxylic acids), peptides, aminoacids, sugars, antocyanines, nucleic acids and other compounds with chemical, biochemical or pharmacological relevance.

8. The use of compounds according to claims 1 to 6, in the case of being liquids, in pervaporation processes, liquids have been defined as all the compounds according to claims 1 to 6 in a liquid state at temperature between -50° C and 150 ° C, preferably between 0 ° C and 80 ° C; pervaporation has been defined as a separation process based on the molecular interaction between the feed components and the dense, non-porous membrane polymer, where any solute having diffused toward the membrane downstream surface is ideally instantaneously desorbed and subsequently removed from the downstream side of the membrane; this can be achieved either by applying a vacuum (vacuum pervaporation), or by passing an inert gas over the membrane downstream surface (sweeping-gas pervaporation).

9. The use of compounds according to claims 1 to 6 in biphasic extraction.

10. The use of compounds according to claims 1 to 6 in dissolution of carbohydrates (such as cellulose, glucose, maltose and dextrose), of carbon nanotubes, of metals, of metal salts and metal nanoparticles, supercritical CO2, and other compounds with chemical, biochemical or pharmacological relevance.

11. The use of compounds according to claims 1 to 6 in synthetic transformations including chemical catalysis, biocatalysis or as chiral auxiliaries; and electrochemical transformation including their potential use as electrolyte and/ or in fuel cells.
